# EUROPÄISCHE PATENTSCHRIFT

(11) **EP 2 004 037 B1**
(45) Veröffentlichungstag und Bekanntmachung des Hinweises auf die Patenterteilung: **12.09.2018**
(21) Anmeldenummer: 07722172.9
(22) Anmeldetag: 04.04.2007
(51) Int. Cl.: A61B 5/00

(54) **VORRICHTUNG ZUR BESTIMMUNG EINES VERGLEICHSWERTES VON BIODATEN SOWIE ZUR ERMITTLUNG VON BIODATEN**
DEVICE FOR DETERMINING A COMPARISON VALUE OF BIODATA AND FOR RECORDING BIODATA
DISPOSITIF POUR LA DETERMINATION D'UNE VALEUR COMPARATIVE DE DONNÉES BIO AINSI QUE POUR LA DETERMINATION DE DONNEES BIO

(30) Priorität: 07.04.2006 DE 102006018041; 07.04.2006 DE 102006018040
(43) Veröffentlichungstag der Anmeldung: 24.12.2008
(73) Patentinhaber: Löwenstein Medical Technology S.A., 2557 Luxembourg (LU)
(72) Erfinder: SCHWAIBOLD, Matthias, 76137 Karlsruhe (DE); SOMMERMEYER, Dirk, 76185 Karlsruhe (DE); SCHÖLLER, Bernd, 76135 Karlsruhe (DE)
(74) Vertreter: Klickow & Wetzel PartGmbB
(86) Internationale Anmeldenummer: PCT/DE2007/000614
(87) Internationale Veröffentlichungsnummer: WO 2007/115553

(56) Entgegenhaltungen:
- EP-A- 1 440 653
- EP-A- 1 704 817
- WO-A-02/15784
- US-B1- 6 216 032

## Beschreibung

Die Erfindung ist in den Ansprüche definiert. Die Erfindung betrifft eine Vorrichtung zur Bestimmung eines Vergleichswertes von Biodaten (Körperparameter) eines Lebewesens zur Ermittlung eines individuellen Risikos, bestehend aus mindestens einem Sensor zur nicht invasiven Messung von mindestens zwei Signalen ausgewählt aus der Gruppe: CWF (continous wave fluctuation), Sp02, Pulsfrequenz, Herzfrequenz, PTT (pulse transit time) und einer an den Sensor angeschlossenen Auswertungseinrichtung, wobei die Auswertungseinrichtung wenigstens einen Analysator aufweist, der durch Signalanalyse definierbare Signalbereiche ermittelt, wobei eine Vergleichseinrichtung die Signalbereiche unter Berücksichtigung weiterer Parameter wie bestehende Biodaten des Lebewesens und/oder statische Datensätze abstimmt und das Ergebnis als Index-Wert ausgebbar ist, wobei der Sensor das Rohplethysmogramm als ein Signal aufzeichnet, aus welchem die Auswertungseinrichtung einen CWP (continous wave Parameter) extrahiert, welcher über die Zeit betrachtet als CWF-Signal berechnet wird.

Bevorzugt wird ein Plethysmogramm verwendet, das beispielsweise mit einem Pulsoximeter aufgezeichnet wird. Aus dem Plethysmogramm werden verschiedene CWP (continous wave parameter) extrahiert. CWP sind verschiedene, fluktuierende Parameter, die jeweils eine Eigenschaft des Plethysmogramms beschreiben. Beispielsweise können folgende Größen als CWP berechnet werden: die Pulswellenamplitude, das Integral über bestimmte Abschnitte des Plethysmogramms, das Verhältnis verschiedener Integrale über verschiedene Abschnitte des Plethysmogramms, ein mit der Atmung korrelierender Anteil des Plethysmogramms, der Anstiegswinkel jeder Pulswelle, der Abfallswinkel jeder Pulswelle, das Verhältnis aus Anstiegs- zu Abfallwinkel, die Dauer eines Pulswellenanstiegs, die Dauer eines Pulswellenabfalls, das Verhältnis der Anstiegs- zur Abfallsdauer, das Pulswellenmaximum, das Pulswellenminimum, eine mit der PTT (Puls Transit Time) in Zusammenhang stehende Größe. Erfindungsgemäß soll jeglicher Parameter, der eine Eigenschaft eines Abschnitts des Plethysmogrammsbeschreibt, als CWP darstellbar sein.

Betrachtet man CWP über der Zeit, so kann daraus ein CWF- Signal (continous wave fluctuation) berechnet werden, das Schwankungen (= Fluktuationen) unterliegt, die für die Signalauswertung relevant sind. Das CWF-Signal enthält also Informationen über die Schwankungen (= Fluktuationen) des Plethysmogramms, bzw. der daraus abgeleiteten CWP. Es ist denkbar eine der folgenden Größen als CWF-Signal darzustellen: die Pulswellenamplituden, mit dem Integral über einen Abschnitt des Plethysmogramms in Zusammenhang stehende Parameter, Verhältnisses verschiedener Integrale über verschiedene Abschnitte des Plethysmogramms, mit der Atmung korrelierende Anteile des Plethysmogramms, Anstiegswinkel der Pulswellen, Abfallswinkel der Pulswellen, Verhältnisse aus Anstiegs- zu Abfallwinkel, Dauern von Pulswellenanstiegen, Dauern von Pulswellenabfällen, Verhältnisse von Anstiegs- zu Abfallsdauem, die Einhüllende der Pulswellenma-xima oder Pulswellenminima, mit der PTT (Puls Transit Time) in Zusammenhang stehende Größen, die Mittellinie des Plethysmogramms, die Pulsfrequenz. Es ist jedoch ebenfalls daran gedacht die PTT oder andere Signale, die Schwankungen unterliegen, zur Ermittlung einer CWF zu nutzen. Erfindungsgemäß soll jegliche Schwankung des Plethysmogramms, jeder CWP und jede Kombination verschiedener CWP als CWF- Signal darstellbar sein. Es besteht das Problem einen Risiko-Index zu ermitteln, der zur Unterstützung einer Diagnose, Einstellung und Kontrolle von Lebewesen dient und durch stationäre und ambulante Untersuchungen feststellbar ist.

Bei vielen Erkrankungen bestehen Abhängigkeiten zu kardiovaskulären Erkrankungen beziehungsweise Wechselwirkungen, welche die Lebensqualität des Patienten beeinflussen können. Derartige Phänomene lassen sich bislang lediglich statistisch für ein Patientenkollektiv ermitteln, jedoch nicht für einen bestimmten individuellen Patienten erfassen.

Es sind bereits verschiedene Messverfahren bekannt, um einzelne Parameter zu erfassen, die sich auf die autonome Regulation des kardiovaskulären Systems beziehen. Derartige Messverfahren werden beispielsweise in der US 5,862,805, der WO 91/11956, der US 2002-0029000, der WO 02/067776 sowie der EP 0 995 592 beschrieben. Es sind bislang jedoch keine Verfahren und keine Vorrichtungen bekannt geworden, die eine umfassende Auswertung der einzelnen Messwerte zur umfassenden Berücksichtigung der Einzelfaktoren bei der Ermittlung eines individuellen Risiko-Index betreffen.

In der US 6,216,032 werden ein Verfahren und eine Vorrichtung beschrieben, die eine automatische Diagnose von Erkrankungen basierend auf der messtechnischen Erfassung der Herzratenvariabilität ermöglichen.

In der EP 1 440 653 wird eine Vorrichtung beschrieben, bei der basierend auf der messtechnischen Ermittlung einer Blutkomponente und einer anschließenden Signalverarbeitung eine automatisierte Bewertung von Stress-Zuständen des menschlichen Körpers ermöglicht.

In der EP 1 704 817 A wird eine Vorrichtung zur Ermittlung von klinischem Stress beschrieben. Es wird hier unter Verwendung der Fotoplethysmografie während einer Anästhesie Pulswellensignale aufgezeichnet und analysiert. Das Messergebnis wird zur Steuerung einer Medikamentenpumpe verwendet.

Insbesondere existieren keinerlei Vorrichtungen, die aus der Kombination der von ihnen gemessenen Parameter Vorhersagen über das individuelle Risiko ermöglichen, aufgrund der vorliegenden Erkrankung, welche möglicher- aber nicht notwendigerweise mit derselben Vorrichtung diagnostiziert wird, Folgeerkrankungen zu entwickeln, welche die Lebensqualität bzw. Lebenserwartung beeinträchtigen.

Ebenso existieren keinerlei Vorrichtungen, die aus der Kombination der von ihnen gemessenen Parameter Aussagen darüber ermöglichen, aus den für die Erkrankung, welche möglicher- aber nicht notwendigerweise mit derselben Vorrichtung diagnostiziert wird, existierenden Therapieverfahren und Therapiedosierungen qualitativ und/oder quantitativ geeignet sind.

Die erfindungsgemäße Vorrichtung sowie das Verfahren beziehen sich ebenfalls auf ein Modulsystem zur Ermittlung von Biodaten eines Lebewesens, bestehend aus mindestens einer Sensoreinrichtung zur nicht invasiven Messung von mindestens zwei Signalen.

Hierdurch sollen beispielsweise schlafbezogene Erkrankungen und/oder Herz-Kreislauf-Erkrankungen und/oder Stoffwechsel- Erkrankungen ermittelt und eine Diagnose unterstützt werden. Die Ermittlung schlafbezogener Störungen bzw. Erkrankungen erfolgt derzeit immer durch einen Facharzt und zumeist während der Nacht in einem Schlaflabor. Üblicherweise wird dazu ein Polysomnographiegerät verwendet. Aufgrund der geringen Anzahl an ausgebildeten Fachärzten und der geringen Anzahl an Schlaflaboren müssen viele Patienten häufig lange auf einen Diagnosetermin warten bzw. eine Diagnose wird gar nicht erst gestellt.

Aufgabe der vorliegenden Erfindung ist daher, eine Vorrichtung bereit zu stellen, die eine einfache, schnelle und preiswerte Diagnosestellung ermöglicht. Darüber hinaus soll durch Auswertung von Parametern der Pulswelle eine erweiterte Aussage, gegenüber der bisherigen Polysomnographie, zur Verfügung gestellt werden. Diese erweiterte Aussage soll insbesondere die Aktivität und Beurteilung des autonomen Nervensystems betreffen.

Insbesondere existieren keinerlei Vorrichtungen mit weniger Eingangskanälen als Polysomnographiegeräte, die eine Diagnosestellung erlauben, die der eines Polysomnographiegerätes vergleichbar bzw. in Bezug auf spezielle Fragestellungen überlegen ist.

Die Lösung dieser Aufgabe erfolgt erfindungsgemäß durch die Merkmale von Patentanspruch 1.

Es wird hierbei mindestens ein Sensor zur nicht invasiven Messung von mindestens zwei Signalen ausgewählt aus der Gruppe: CWF (continous wave fluctuation), Sp02, Herzfrequenz, PTT (pulse transit time) an eine Auswertungseinrichtung angeschlossen, die Auswertungs-einrichtung weist wenigstens einen Analysator auf, der Analysator ermittelt durch Signalanalyse definierbare Signalbereiche, eine Vergleichseinrichtung analysiert die Signalbereiche unter Berücksichtigung weiterer Parameter und das Ergebnis der Analyse wird als Index-Wert an eine angeschlossene Ausgabeeinrichtung übermittelt.

Ziel der Erfindung ist also nicht die Diagnose einer Erkrankung und auch nicht die reine Ermittlung der Häufigkeit und Stärke des Auftretens bestimmter pathophysiologischer Ereignisse. Vielmehr wird anhand der erfassten Signale ermittelt, wie hoch das individuelle Risiko der untersuchten Person ist, Folgeerkrankungen zu erleiden, welche die Lebensqualität bzw. Lebenserwartung beeinträchtigen. Ebenso kann anhand des ermittelten Risikos abgeleitet werden, welche Therapieform und Therapiedosierung für die Person optimal ist. Außerdem kann der Erfolg einer bereits bestehenden Therapie gemessen werden.

Durch eine Auswertung von wenigstens zwei der folgenden Signale ausgewählt aus der Gruppe: CWF (continous wave fluctuation), SpO2, Herzfrequenz, PTT (Puls - Transit - Zeit) beispielsweise durch Anwendung der Photoplethysmographie, ist es möglich, die Wechselwirkungen einer Vielzahl von physiologschen und pathophysiologischen Prozessen zu bestimmen. Zusätzlich ist es möglich, dass unter Verwendung einer gegenüber dem Stand der Technik erheblich reduzierten Anzahl von Messsensoren, nämlich nur einem Messsensor beispielsweise in Form eines Pulsoximetrie-Sensors, vergleichbare Aussagen zu bereits bekannten Verfahren getroffen werden, beispielsweise hinsichtlich von Atmungsstörungen, Schlafstörungen, Diabetes, Entzündungseraktionen, Gefäßzuständen und Herz-Kreislauferkrankungen.

Für einen Patienten sind somit individuelle Aussagen zu seinem Gesundheitszustand möglich bezogen auf:
- Kardiovaskuläres- Risiko
- Stress
- Diabetes
- Entzündungen
- autonome Funktionsstörungen und damit zusammenhängende Erkrankungen
- Risiko-Index (Prozentual)
- Klassen Risiko-Gruppen
- Differenzierung der Risiko Faktoren / Behandlung erforderlich ja/nein

Eine weitere Aufgabe der Erfindung ist es, eine modulare Vorrichtung bereit zu stellen, die es ermöglicht, bedarfsabhängig ergänzende Module schnell und einfach zu adaptieren.

Ebenso existieren keinerlei Vorrichtungen, die bedarfsgerecht adaptierbar sind, vielmehr sind gegenwärtige Polysomnographiegeräte immer mit allen Kanälen bestückt, auch wenn für die aktuelle Untersuchung nur wenige Kanäle ausreichend und sinnvoll wären.

Aufgabe der vorliegenden Erfindung ist es daher auch, eine Vorrichtung der einleitend genannten Art derart zu konstruieren, dass eine individuelle Anwendbarkeit für einen Patienten ermöglicht wird, in dem Sinne, dass für den Patienten nur die spezifischen Module zur Analyse benutzt werden, die tatsächlich notwendig sind. Für weitergehende Analysen sind schnell und einfach weitere Module an das Grundmodul adaptierbar. Ein besonderer Vorteil der erfindungsgemäßen Vorrichtung ist, dass auch medizinische Laien, zumindest aber medizinisches Personal oder fachfremde Ärzte ohne weiteres in der Lage sind, dem Patienten die erfindungsgemäße Vorrichtung schnell und korrekt anzulegen.

Diese Aufgabe wird erfindungsgemäß dadurch gelöst, dass ein Grundmodul Schnittstellen zur Adaption ergänzender einzelner Module für eine Ermittlung weiterer Biodaten, wie EKG, Herzfrequenz, Atemfluss, PTT (pulse transit time) aufweist.

Bevorzugt wird ein Plethysmogramm beispielsweise mit einem Pulsoximeter und/oder einem mehrwellenlängen Pulsspektrometer (gemäß DE 102005020022 A1, DE 10213692 A1, DE 10321338 A1) aufgezeichnet. Die Begriffe Pulsoximeter und/oder einem Pulsspektrometer werden hier synomym verwendet. Das Pulsoximeter und/oder das Pulsspektrometer ermitteln, unter Verwendung von zumindest zwei Wellenlängen ausgewählt aus dem Bereich 400 bis 2500 nm, zumindest die folgenden Parameter: Pulsfrequenz, Plethysmogramm und Sauerstoffsättigung (SpO2 und/oder SaO2). Im Folgenden werden die Begriffe SpO2 und SaO2 daher synonym verwendet.

Aus dem Plethysmogramm wird zumindest ein CWP (continous wave parameter) extrahiert, bevorzugt werden zumindest zwei oder mehr CWP extrahiert.

Erfindungsgemäß können verschiedene Signale kombiniert werden, um relevante Schwankungen der Pulswelle zu detektieren und für die Auswertung heranzuziehen.

Beispielsweise ist daran gedacht, eine Unterscheidung von obstruktiven und zentralen Atmungsstörungen anhand von Mustern vorzunehmen, die in einem aus dem Plethysmogramm abgeleiteten CWF-Signal detektiert werden. Dabei weisen die charakteristischen Muster Frequenzanteile auf, die mit der Atemfrequenz in Verbindung stehen. Eine Signalauswertung über eine vorgebbare Zeitspanne wird dadurch unterstützt, dass der Sensor mit einer ersten Speichereinheit zur Abspeicherung eines erfassten Messsignals verbunden ist.

Zur Durchführung einer Verlaufsanalyse des erfassten Signals wird vorgeschlagen, dass die Auswertungseinrichtung einen Verlaufsanalysator zur Analyse des zeitlichen Verlaufes des Signals aufweist.

Zur weiteren Verbesserung der Auswertungsmöglichkeiten wird vorgeschlagen, dass die Vergleichseinrichtung mit einer zweiten Speichereinheit zur Abspeicherung des errechneten Index-Werts versehen ist.

Ein typischer Auswertungsablauf erfolgt dadurch, dass der Amplitudenverlauf des Messsignals ausgewertet wird.

Ebenfalls ist daran gedacht, dass eine Steigung des Messsignals ausgewertet wird.

Gemäß einer weiteren Ausführungsvariante ist vorgesehen, dass eine Frequenz des Messsignals ausgewertet wird.

Hinsichtlich der Aussageempfindlichkeit erweist es sich insbesondere als vorteilhaft, dass eine Änderungsintensität des Messsignals ausgewertet wird.

Eine umfassende Signalauswertung kann dadurch erfolgen, dass eine Mustererkennung durchgeführt wird.

Eine weitere Signalauswertung kann dadurch erfolgen, dass eine periodische und / oder transiente Signalanalyse durchgeführt wird.

Eine ergänzende Signalauswertung kann dadurch erfolgen, dass eine Frequenz-Analyse durchgeführt wird.

Eine alternative Signalauswertung kann dadurch erfolgen, dass eine Analyse der Steigung durchgeführt wird.

Eine Signalauswertung kann dadurch erfolgen, dass Histogramme und/oder Verteilungen und/oder Ableitungen gebildet werden.

Eine alternative Signalauswertung kann dadurch erfolgen, dass Schwellenwerte verglichen und/oder korreliert werden.

Eine Erstellung einer Hierarchie der Signale und/oder eines Entscheidungsbaumes kann die Signalauswertung weiter unterstützen.

Eine Möglichkeit zur Signalerfassung besteht darin, dass ein CWF (continous wave fluctuation)-Signal ausgewertet wird.

Beispielsweise werden die Amplituden (51) des Plethysmogramms (49) zur Ermittlung eines CWF-Signals verwendet. Das CWF (50) stellt in diesem Fall die Amplitudenhöhen der einzelnen Pulswellen des Plethysmogramms (49) dar.

Es ist jedoch ebenfalls daran gedacht, die PTT oder andere Signale, die Schwankungen unterliegen, zur Ermittlung einer CWF zu nutzen.

Erfindungsgemäß können verschiedene Signale kombiniert werden, um relevante Schwankungen der Pulswelle zu detektieren und für die Auswertung heranzuziehen.

Eine andere Möglichkeit zur Signalerfassung besteht darin, dass ein Sauerstoffsättigungs-Signal ausgewertet wird.

Eine weitere Möglichkeit zur Signalerfassung besteht darin, dass ein Pulsfrequenz- und/oder Herzfrequenz-Signal ausgewertet wird.

Eine andere Möglichkeit zur Signalerfassung besteht darin, dass eine PTT (pulse transit time) ausgewertet wird.

Eine ergänzende Möglichkeit zur Signalerfassung besteht darin, dass ein EEG-Signal ausgewertet wird.

Ebenfalls ist daran gedacht, dass ein EKG-Signal ausgewertet wird.

Ebenfalls ist daran gedacht, dass ein EMG-Signal ausgewertet wird.

Eine weitere Messvariante besteht darin, dass eine Sauerstoffsättigung des Blutes ausgewertet wird.

Eine weitere Messvariante besteht darin, dass eine Hämoglobinkonzentration des Blutes ausgewertet wird.

Zur Erfassung von respiratorischen Parametern ist vorgesehen, dass ein Atmungsverlauf ausgewertet wird.

Zur Erfassung von weiterer Parameter ist vorgesehen, dass ergänzend auch Schnarchen, Arousals, Blutdruck, CO2, Schlafstadien, Hautleitwert, Schlaftiefe, Schlaffragmentierung, Aktivität des Parasympathikus absolut oder im Verhältnis zum Sympathikus, Gefäßcompliance ausgewertet werden können.

Ein einfach zu realisierendes Messprinzip besteht darin, dass eine optische Dichte mindestens eines Körperbereiches ausgewertet wird.

Gemäß einem typischen Auswertungsverfahren ist vorgesehen, dass eine Signalauswertung im Hinblick auf vorliegende periodische Signalanteile durchgeführt wird.

Eine Auswertung besonders aussagefähiger Signalverläufe erfolgt dadurch, dass eine Analyse hinsichtlich einer maximalen Signaländerung durchgeführt wird.

Insbesondere erweist es sich als vorteilhaft, dass der Index einer kumulativen autonomen Ruheintensität der Regulation des kardiovaskulären Systems zugeordnet ist.

Die Erfassung besonders aussagekräftiger Ereignisse wird dadurch erreicht, dass eine Signalanalyse hinsichtlich eines Zeitraumes einer Aktivierung des autonomen Systems durchgeführt wird.

Eine nochmalige Steigerung der Aussagequalität kann dadurch erreicht werden, dass bei der Auswertung von Zeiträumen der Aktivierung des autonomen Systems mindestens ein weiterer Parameter ausgewertet wird. Die Steigerung der Aussagequalität kann dadurch erreicht werden, dass die Änderung verschiedener erfasster oder aus den erfassten Parametern abgeleiteter Parameter zum Zeitpunkt einer Aktivierung des autonomen Systems in Stärke, Typ und zeitlicher Abfolge verglichen wird.

Zur Eliminierung von Störungen oder singulären Ereignissen wird vorgeschlagen, dass bei der Ermittlung des Index die kumulative Anzahl und die Intensität der Aktivierungszeiträume des autonomen Systems berücksichtigt wird.

Ein einfacher Messaufbau wird dadurch unterstützt, dass vom Sensor die Herzfrequenz erfasst wird.

Insbesondere ist daran gedacht, dass vom Sensor die Variabilität der Herzfrequenz erfasst wird.

Darüber hinaus ist es auch möglich, dass vom Sensor die PTT erfasst wird.

Darüber hinaus ist es auch möglich, dass vom Sensor ein CWF erfasst wird.

Alternativ ist es möglich, dass vom Sensor die Pulsamplitude erfasst wird.

Zur Störungsunterdrückung trägt es bei, dass vor der Auswertung der erfassten Parameter eine Artefakterkennung bzw. -beseitigung durchgeführt wird.

Eine Erkennung von kurzfristigen Signaländerungen wird dadurch unterstützt, dass ein innerhalb eines vorgebbaren Zeitintervalls auftretender Maximalwert des vom Sensor erfassten Messsignals ausgewertet wird.

Ein einfach anwendbares Messverfahren besteht darin, dass die Signalerfassung unter Anwendung der Photopletysmographie durchgeführt wird.

Eine Steigerung der Systemempfindlichkeit kann dadurch erreicht werden, dass die Signalauswertung innerhalb mindestens eines vorgebaren Frequenzbandes durchgeführt wird.

Zur Berücksichtigung von Wechselwirkungen wird vorgesehen, dass von der Auswertungseinheit mindestens ein weiterer Körperparameter ausgewertete wird.

Beispielsweise ist daran gedacht, dass als weiterer Körperparameter das Alter des Lebewesens ausgewertet wird.

Ebenfalls ist es möglich, dass als weiterer Körperparameter das Geschlecht des Lebewesens ausgewertet wird.

Alternativ oder ergänzend ist es auch möglich, dass als weiterer Körperparameter das Gewicht des Lebewesens ausgewertet wird. Alternativ oder ergänzend ist es auch möglich, dass als weiterer Körperparameter ein oder mehrere bereits bekannte Risikofaktoren beispielsweise für eine kardiovaskuläre Erkrankung ausgewertet werden.

Alternativ oder ergänzend ist es auch möglich, dass als weiterer Körperparameter ein oder mehrere bereits bekannte Faktoren, die die autonome Regulation beeinflussen, ausgewertet werden, insbesondere die Medikation.

Alternativ oder ergänzend ist es auch möglich, dass als weiterer Körperparameter ein oder mehrere zusätzlich erfasste Parameter ausgewertet werden, z. B. die arterielle Sauerstoffsättigung.

Eine Berücksichtigung von zeitlich zurückliegenden Ereignissen kann dadurch erfolgen, dass als weiterer Körperparameter die Krankenhistorie des Lebewesens ausgewertet wird.

Eine weitere Steigerung der Aussagequalität kann dadurch erreicht werden, dass als weiterer Körperparameter die Medikation des Lebewesens ausgewertet wird.

Eine nochmalige Steigerung der Aussagequalität kann dadurch erreicht werden, dass als weiterer Parameter Vergleichswerte weiterer Personen ausgewertet werden.

Erfindungsgemäß werden, beispielsweise mit einem Pulsoximeter-Sensor, zumindest folgende Signale ermittelt: continous wave fluctuation: CWF , SpO2, Pulsfrequenz.

Ergänzend werden, durch Hinzunahme geeigneter Sensoren, respiratorische Signale wie Fluss, Druck oder Schnarchen, Herzfrequenz, PTT (Puls - Transit - Zeit) registriert und analysiert. Ebenfalls ist daran gedacht EKG-Signale, EEG-Signale, EMG-Signale für die Auswertung zu verwenden. Weiterhin können Blutdruck und CO2-Konzentration aufgezeichnet und für die Auswertung herangezogen werden.

Die Auswertung der Signale erfolgt entsprechend wenigstens einer der folgenden Methoden:
- Mustererkennung
- Signalanalyse (harmonisch/ transient)
- Frequenz-Analyse
- Analyse der Steigung
- Histogramme, Verteilungen, Ableitungen, Integrationen
- Kombination (statistisch) verschiedener Faktoren, Messwerte, ermittelte Werte,
- Ereignis Daten, langfristiger Trend
- Schwellwert-Vergleich,
- Korrelation
- Hierarchie der Signale
- Entscheidungsbaum
- Digitale Filterung
- Wavelet-Analyse
- Zwischenspeicherung von Signal-, CWP- oder CWF-Abschnitten
- Entropie, Standardabweichung
- Verfahren der Chaos-Theorie

Im Ergebnis ermittelt die erfindungsgemäße Vorrichtung einen für den Patienten spezifischen Risiko-Index. Dieser kann beispielsweise prozentual angegeben werden. Bevorzugt wird dazu die medizinische Historie des Patienten zusammen mit den aktuellen Messdaten zur Ermittlung des Risiko-Index berücksichtigt. Als Datenbasis ist im Bereich der erfindungsgemäßen Vorrichtung zumindest ein auslesbarer Speicher verwendet.

Weiterhin sind erfindungsgemäß folgende Ausgabeoptionen alternativ und/oder ergänzend vorgesehen:
- Angabe des kardiovaskulären- Risikos/ Stress
- Angabe des Risikos an Diabetes zu erkranken
- Angabe des Risikos an Entzündungen zu erkranken
- Angabe autonomer Funktionsstörungen und damit zusammenhängender Erkrankungen
- Angabe von Klassen und / oder Risiko-Gruppen

Erfindungsgemäß ist außerdem eine Differenzierung der Risiko-Klassen vorgesehen. Aufgrund der Ausgabe der erfindungsgemäßen Vorrichtung kann beispielsweise ein Arzt gezielt eine Behandlung einleiten.

Gemäß einer bevorzugten Erfindungsvariante wird aus mindestens einem Meßsignal zunächst der CWP errechnet. Anschließend wird die CWF wahlweise aus dem ursprünglichen Meßsignal, dem CWP oder sowohl aus dem Meßsignal als auch aus dem CWP bestimmt.

Die ermittelte CWF kann beispielsweise im Rahmen eines automatisierten Diagnosesystems genutzt werden. Darüber hinaus ist aber auch daran gedacht, die CWF unmittelbar für eine Gerätesteuerung auszuwerten.

Die CWF kann zur Durchführung einer weitergehenden Analyse mit abgespeicherten Werten oder Verläufen oder mit aktuellen Meßwerten verglichen werden.

Im folgenden ist die Erfindung in Anwendungsbeispielen erläutert. Es zeigen:
- Fig. 1: eine Vorrichtung zur Ermittlung von Werten in mehreren Ansichten,
- Fig. 2: adaptierbare Sensoren der Vorrichtung,
- Fig. 3: Peripheriegeräte der Vorrichtung,
- Fig. 4: Gurte zur Befestigung der Vorrichtung,
- Fig. 5: Anwendung mit adaptierten Sensoren,
- Fig. 6: eine Vorrichtung mit angeschlossenem Rechner und Sichtgerät,
- Fig. 7: ein Diagramm einer Sensorregistrierung mit einem Sensorrohsignal und ein extrahiertes Signal (CWF-Signal),
- Fig. 8: ein Plethysmogramm und ein extrahiertes Signal (CWF-Signal) zur Amplitudenhöhendarstellung,
- Fig. 9: eine Armmanschette mit aufgenommenen Grundmodul und zwei ergänzenden Modulen und

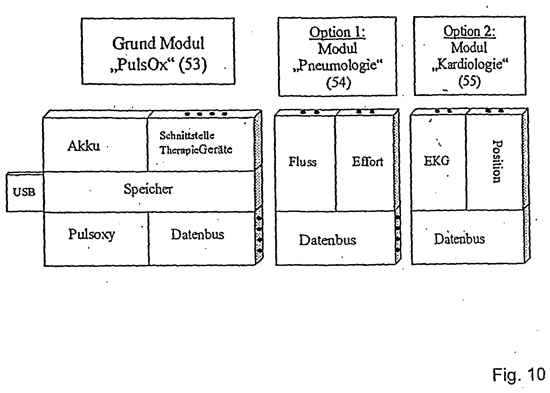

- Fig. 10: eine Prinzipdarstellung mit einem Grundmodul und zwei ergänzenden zugeordneten Modulen.

Die Vorrichtung ermöglicht, in einem bevorzugten Ausführungsbeispiel, eine schnelle Ermittlung des Risiko-Index dadurch, dass mit Hilfe eines Pulsoximetrie-Sensors zumindest drei Signale, nämlich CWF, Sauerstoffsättigung des Blutes und Pulsfrequenz im wesentlichen gleichzeitig ermittelt werden. Durch eine Mustererkennung werden die Signale analysiert und mit gespeicherten Werten verglichen. Insbesondere wird die Veränderung der Amplitude der Pulswelle analysiert. Das Ergebnis des Vergleichs ergibt einen für den Patienten spezifischen Index-Wert, der geeignet ist, eine Aussage über das Risiko einer Herz-Kreislauferkrankung zu ermöglichen. Die erfindungsgemäße Vorrichtung ist klein und tragbar. Die Energieversorgung erfolgt alternativ über Akkumulatoren / Batterien und/oder über ein Netzkabel.

Die gemessenen Daten werden im Gerät auf einer CompactFlash® Card gespeichert, sowie online über Kabel oder optional drahtlos an den PC übertragen. Für den ambulanten Einsatz können die im Gerät gespeicherten Daten entweder über eine USB-Schnittstelle an den PC übertragen oder durch Auslesen der CompactFlash® Card über ein Lesegerät in die Software eingelesen werden.

Die erfindungsgemäße Vorrichtung zur Ermittlung und Analyse von Biodaten eines Lebewesens besteht aus einem Grundmodul mit einer Energieversorgung und einer Speichereinheit und mindestens einer Sensoreinrichtung zur nicht invasiven Messung von zumindest einem Meßsignal das die Herztätigkeit und/oder die Atmungstätigkeit repräsentiert. Die Sensoreinrichtung kann beispielsweise ausgewählt sein aus der Gruppe: EKG, Blutdruckmanschette, Pulsoximeter, Impedanzsensor, Dopplersensor.

Das Meßsignal, welches die Herztätigkeit und/oder die Atmungstätigkeit repräsentiert ist ausgewählt aus der Gruppe: Pulsfrequenz, Plethysmogramm, Sauerstoffsättigung, Atmungssignal, Herzsignal. An die Sensoreinrichtung angeschlossenen ist eine Analyseeinrichtung zur Extraktion von zumindest einem CWP (continous wave parameter) aus dem Meßsignal, und/oder eine Einrichtung zur Ermittlung von zumindest einer CWF (continous wave fluctuation) wobei die CWF (conti-nous wave fluctuation) bevorzugt aus dem CWP und/oder dem Meßsignal ermittelt wird.

Ein Klassifikator vergleicht zumindest eine CWF-Information und/oder daraus abgeleitete Größen mit hinterlegten Daten um daraus physiologische / pathophysiologische Ereignisse zu identifizieren. Solche Ereignisse sind beispielsweise ein Apnoe, wobei erfindungsgemäß zwischen zentralen und obstruktiven Apnoen unterschieden werden kann.

In einem anderen Ausführungsbeispiel vergleicht ein Klassifikator zumindest eine CWF-Information und/oder daraus abgeleitete Größen mit anderen Meßsignalen und/oder CWP vergleicht um daraus physiologische / pathophysiologische Ereignisse zu identifizieren.

Zumindest ein Teil der Ergebnisse der Analyseeinrichtung und/oder zumindest ein Teil der Ergebnisse des Klassifikators werden im wesentlichen unmittelbar akustisch und/oder graphisch, bevorzugt über ein Display, ausgegeben.

Erfindungsgemäß werden zur Identifikation physiologischer Ereignisse zumindest zwei Signale, die in zeitlichem Zusammenhang stehen, ausgewertet.

Die erfindungsgemäße Vorrichtung kann zur Messung weiterer Signale um ergänzende Module erweitert werden. Dazu werden die Module bevorzugt im Sinne eines "plug and play" adaptiert. Bevorzugt werden dazu ergänzende Sensoreinrichtungen adaptiert.

Dazu ist zumindest eine Schnittstelle im Bereich des Grundmoduls und/oder im Bereich ergänzender Module angeordnet.

Über die Schnittstelle ist auch das Auslesen von Daten und/oder das Steuern von anderen Geräten, insbesondere Therapiegeräten möglich.

Als adaptierbare Sensoreinrichtungen sind alternativ oder ergänzend zumindest folgende vorgesehen: EKG-, EMG-, EOG-, EEG-, Pulsoximetrie-, Blutdruck-, Impedanzmessungs-, Ultraschall-, Doppler-, CO2-, Atemfluss-, Schnarch-, Mund-, Thorax-, Abdomen-, Lage-Sensor.

Die Sensoreinrichtungen können in Form eines Moduls ergänzt werden kann welches beispielsweise zur Ermittlung respiratorischer Parameter, wie Atemfluss, PTT, Bewegungssignale, Atemanstrengung ausgebildet ist.

Die Sensoreinrichtungen können auch in Form eines Moduls ergänzt werden, welches zur Ermittlung kardiologischer Parameter, wie EKG, Herz-frequenz, ausgebildet ist.

Zur Eingabe von Patienten-Daten wie beispielsweise Alter, Geschlecht, Gesundheitszustand, ist eine Vorrichtung zur Dateneingabe vorgesehen.

Ein Display ist als ein Anzeigemittel für Analyseergebnisse vorgesehen. Zudem sind akustische Alarme vorgesehen.

Erfindungsgemäß wird zumindest das Grundmodul mit Befestigungs-Mitteln, wie beispielsweise Gurten, lösbar am Körper eines Patienten fixiert.

In einer anderen Ausführungsform besteht die Vorrichtung zur Ermittlung und Analyse von Biodaten eines Lebewesens, aus mindestens einer Sensoreinrichtung zur nicht invasiven Messung von mindestens zwei Signalen, wie Plethysmogramm und daraus abgeleiteten CWF-Signalen (conti-nous wave fluctuation), SpO2, Pulsfrequenz und einer an die Sensoreinrichtung angeschlossenen Analyseeinrichtung zur Analyse transienter und/oder periodisch wiederkehrender Muster der gemessenen Signale. Ergänzend ist ein Modul zur Auswertung von solchen Informationen, die mit der Frequenz oder Amplitude der Signale oder der aus ihnen abgeleiteten Parameter in Zusammenhang stehen, vorgesehen.

In einer ergänzenden Ausführungsform besteht die Vorrichtung zur Ermittlung und Analyse von Biodaten eines Lebewesens aus einem Grundmodul mit einer Energieversorgung und einer Speichereinheit und mindestens einer Sensoreinrichtung zur nicht invasiven Messung von zumindest einem Meßsignal, das die Atmungstätigkeit repräsentiert, beispielsweise Sauerstoffsättigung, Atmungssignal, Drucksignal, Flusssignal und mindestens einer weiteren Sensoreinrichtung zur nicht invasiven Messung von zumindest einem Meßsignal, das die Herztätigkeit repräsentiert, beispielsweise Sauerstoffsättigung, Blutdruck, Pulsfrequenz, EKG, Plethysmogramm und einer an die Sensoreinrichtung angeschlossenen Analyseeinrichtung zur Extraktion von zumindest einem CWP (continous wave parameter) aus zumindest einem der Meßsignale.

Beispielsweise lassen sich somit durch Analyse der Meßsignale Rückschlüsse auf Apnoen, Hypopnoen und andere Atmungsstörungen gewinnen. Unter Verwendung von mindestens einer Sensoreinrichtung zur nicht invasiven Messung von zumindest einem Meßsignal, das die Atmungstätigkeit repräsentiert, und mindestens einer weiteren Sensoreinrichtung zur nicht invasiven Messung von zumindest einem Meßsignal, das die Herztätigkeit repräsentiert lassen sich beispielsweise durch Mustererkennung Rückschlüsse auf Erkrankungen des Herz-KreislaufSystems und /oder des Atmungsapparates gewinnen.

Im folgenden werden weitere Ausführungsformen beschrieben.
∘Anwendungsteil bestehend aus:
   - Grund-Modul
   - Kardiologie-Modul
   - Pneumologie-Modul
   - Applikationsteile
   - Batterie/Akku, z.B. LiIon-Akku
   - medizinisch zugelassenes Netzgerät (Sekundärseite) mit Kombikabel zur Ladung des Akkus und zur Datenübertragung der gespeicherten Daten über eine galvanisch getrennte USB-Schnittstelle (Konverterbox) an einen PC
∘PC-Software:
   Die Software kann unter den Betriebssystemen Windows 2000 ab SP 2, Windows XP Professional und Home-Edition, Windows Vista betrieben werden.
∘Nichtmedizinische elektrische Geräte:
   - Netzgerät (Primärseite)
   - USB/TCP/IP Konverter
   - Bluetooth®-USB-Adapter
   - CompactFlash® Card-Lesegerät
   - PC-System (Fremdzubehör).

### Grund-Modul

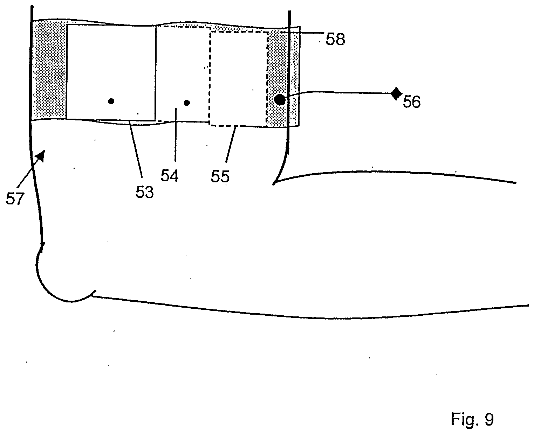

Das Grundmodul besteht aus einem Pulsoximeter, einer möglichen Schnittstelle für die Steuerung anderer Geräte, z.B. Therapiegeräte, möglichen weiteren Schnittstellen für weitere, optionale Sensoren, z.B. Anschluss eines Atemfluss-/Schnarchsensors, einer Kommunikationsschnittstelle wie beispielsweise USB, oder einem drahtlosen Protokoll, einem auslesbaren Speicher, einer Energieversorgung über Batterie / Akkumulator und einem Datenbus Kontroller, als Anschluss für weitere Module und einem Display zur Anzeige von berechneten Indizes, aktuellen Messwerten, aktuellen Batterie/ Akkuladezuständen. Zur Weitergabe der ermittelten Daten ermöglicht das Grundmodul einen bidirektionalen DatenAustausch mit anderen Geräten, beispielsweise mit APAP-Therapiegeräten. Die Fig. 9 zeigt wie das Grund-Modul (53) mit adaptiertem Pneumologie-Modul (54) und adaptiertem Kardiologie-Modul (55) auf einem gemeinsamen Grundträger (58) am Arm (57) eines Anwenders positioniert sind. Eine bidirektionale Datenübertragung ist über das angeschlossene Kabel (56) möglich. Alternative Applikationsorte für die erfindungsgemäße Vorrichtung sind: Finger, Zehe, Nase, Ohr, Stirn.

Die erfindungsgemäße Vorrichtung verfügt über weniger Eingangskanäle als übliche Polysomnographiegeräte und ist damit deutlich preisgünstiger, kleiner, leichter und energiesparender.

### Sensoren:

∘Pulsoximetriesensor zur Erfassung von Plethysmogramm , Sauerstoffsättigung, Pulsfrequenz.

Das Grundmodul kann folgende Werte ermitteln:
- Pulsfrequenz
- Sauerstoffsättigung (SpO2)
- RDI und AHI(jeweils differenziert nach obstruktiv/ zentral)
- Risikowert für obstruktive Atemwegserkrankung
- Risikowert für zentrale Atmungsstörung
- Risikowert für PLM (Periodic Leg Movement)
- Kennwert für Erholungsfunktion des Schlafes
- Autonomer (Micro-)Arousal Index
- Schlafqualitäts-Index
- Index für Respiratorische Ereignisse
- Autonomer Status
- Kennwert für aktuelle Konzentrationsfähigkeit
- Kennwert für hohe Müdigkeit
- Kennwert für hoher Schlafdruck
- Autonome Ruhe
- Index für Herz-Kreislauf-Risiko (Gefäßerkrankung, Rhythmologische Erkrankung, Herzinsuffizienz)
- Leckage, Therapieerfolg
- Index für den Fortschritt einer Erkrankung

### Pneumologie-Modul

Für weitere Analysen kann dieses Modul an den Datenbus des Grundmoduls adaptiert werden. Erfindungsgemäß ist daran gedacht, die Verbindung zwischen Grund- und Pneumologie-Modul als Steckverbindung auszuführen, die einfach und schnell bedienbar ist. Das Pneumologie-Modul kann auch alleine, ohne Grund-Modul, verwendet werden. Das Pneumologie-Modul verfügt wenigstens über eine Effort-Sensorik und eine Sensoreinrichtung zur Ermittlung eines Atemfluss-Signales.

### Mögliche Sensoren:

∘Effort Sensoren
(Thorax- und Abdomenbewegungen);
∘Atemfluss-Schnarch-Sensor (Thermistoren und Mikrofon) ;
∘Atemfluss-Schnarch-Nasenbrille (Drucksensor);
∘Mundthermistor zur Erfassung von Mundatmung bei Therapiekontrolle;
∘Pneumo-T-Adapter zur Erfassung des Atemflows, Schnarchen und xPAP-Druckes (DifferenzDrucksensor) .

Das Pneumologie-Modul kann zusammen mit dem Grund-Modul folgende Werte ermitteln:
- RDI / AHI (Apnoe / Hypopnoe Unterscheidung)
- Arousals die mit einem respiratorischen Ereignis zusammenhängen
- Unterscheidung zwischen RERAS und Arousals die mit Apnoe / Hypopnoe Ereignissen zusammenhängen
- Unterscheidung obstruktiver und zentraler Ereignisse
- Flattening
- Schnarchen
- Upper Airway Resistance Syndrom
- Atemanstrengung, Work of Breathing

### Kardiologie-Modul

Das Kardiologie-Modul ermöglicht eine EKG-Aufzeichnung. Ergänzend kann ein Lagesensor adaptiert werden. Für weitere Analysen kann dieses Modul an den Datenbus des Grundmoduls und / oder des Pneumologie-Moduls adaptiert werden. Erfindungsgemäß ist daran gedacht, die Verbindung zwischen Grund- und / oder Pneumologie-Moduls und Kardiologie -Modul als Steckverbindung auszuführen, die einfach und schnell bedienbar ist. Das Kardiologie - Modul kann auch alleine, ohne Grund-Modul und / oder Pneumologie-Moduls, verwendet werden.

Das Kardiologie-Modul kann folgende Werte ermitteln: EKG
- Herzfrequenz-Variabilität
- Pulstransit Zeit (PTT)
- Lage abhängige Ereignisse

Erfindungsgemäß ist daran gedacht, weitere Module bedarfsabhängig zu adaptieren.

Nachfolgend werden einzelne Gerätekomponenten im Detail beschrieben.

### Sensoren

∘Elektroden für die elektrophysiologischen Kanäle;
∘Effort Sensoren
(Thorax- und Abdomenbewegungen) ;
∘Pulsoximetriesensor zur Erfassung von Sauerstoffsättigung, Pulsfrequenz, Pulswelle, CWF, PTT ∘Atemfluss-Schnarch-Sensor (Thermistoren und Mikrofon) ;
∘Atemfluss-Schnarch-Nasenbrille (Drucksensor);
∘Mundthermistor zur Erfassung von Mundatmung bei Therapiekontrolle;
∘Pneumo-T-Adapter zur Erfassung des Atemflows, Schnarchen und xPAP-Druckes (Drucksensor).

### Konverterbox und Netzgerät

Die Konverterbox dient zur kabelgebundenen Datenübertragung der im Gerät gespeicherten Daten. Die Datenübertragung erfolgt galvanisch getrennt über eine USB-Schnittstelle. Gleichzeitig wird die erfindungsgemäße Vorrichtung über das Netzgerät geladen, bzw. dauerhaft mit Strom versorgt.

### PC-Software

Die PC-Software dient der Erfassung, Speicherung, Verarbeitung, Visualisierung, Auswertung, Dokumentation und der Archivierung von patientenbezogenen Biosignalen. Dies dient zur Unterstützung der Diagnosefindung, Therapieeinstellung und Therapiekontrolle von Schlafstörungen.

### Geräte-Software

Die Geräte-Software dient der Erfassung, Speicherung, Verarbeitung und der Auswertung von Biosignalen. Dies dient zur Unterstützung der Diagnosefindung, Therapieeinstellung und Therapiekontrolle von Schlafstörungen. Die Geräte-Software kommuniziert über ein gesichertes Datenübertragungsprotokoll mit der PC-Software.

Nichtmedizinische elektrische Geräte
∘Lesegerät zum Auslesen der auf der CompactFlash® Card gespeicherten Daten;
∘Online-Modul zur drahtlosen Datenübertragung (Bluetooth-USB-Adapter);
∘USB - TCP/IP-Konverter;
∘PC-System (Fremdzubehör).

Die erfindungsgemäße Vorrichtung erzeugt Informationssignale (z.B. Akku-Ladezustand), die vom Display und/oder dem PC-System graphisch visualisiert und gespeichert werden. Diese Informationssignale dienen zur Überprüfung der Präsenz der aufzuzeichnenden Signale, sowie der Funktionsüberprüfung des Gerätes. Dadurch werden fehlerhafte Aufzeichnungen vermieden und eine sonst notwendige Wiederholung der Nachtmessung entfällt.

Die automatischen Analysen (CWF, PTT, SpO2, Pulsrate, PLM-, Schnarch-, Schlafstadien-, Arousal- und kardiorespiratorische Analyse) können online im Gerät und/oder offline aus den im PC gespeicherten Signalen erfolgen und unterstützen den Auswerter bei der Diagnose von Schlafstörungen, sowie der Therapieeinleitung und Therapiekontrolle.

Die PC-Software dient der Visualisierung, Auswertung, Dokumentation und patientenbezogenen Archivierung von Langzeituntersuchungen zur Diagnostik von beispielsweise Schlafstörungen, Herz-Kreislauferkrenungen, Diabetes. Dafür wird das System konfiguriert und die übertragenen Daten automatisch offline analysiert. Die Software erlaubt die Eingabe von Bemerkungen durch den Anwender. Eine manuelle Reklassifikation der Analyseergebnisse durch den Auswerter ist möglich.

Der Patient ist nach Einweisung durch Fachpersonal und anhand der Gebrauchsanweisung für den Patienten in der Lage, die Sensoren und das Gerät selbst anzulegen.

Im Falle der online-Analyse im Gerät besteht die Möglichkeit, unmittelbar auf die Analyse-Ergebnisse zu reagieren, beispielsweise ein anderes Gerät fernzusteuern. Es ist daran gedacht, im Falle einer Anästhesie beispielsweise Medikamentendosierer und/oder Beatmungsmaschinen zu steuern, bei Piloten verschiedenster Verkehrsmittel (z.B. Auto, LKW, Bahn, Flugzeug, usw.) aufgrund von Analyse-Ergebnissen (z.B. mangelnde Konzentrationsfähigkeit, hohe Müdigkeit, hoher Schlafdruck) Alarme, Autopilotsysteme oder ähnliches zu aktivieren oder im Falle der Schlafmedizin ein Therapiegerät (z.B. CPAP-Gerät) fernzusteuern.

Grundsätzlich besteht die Möglichkeit, die erfindungsgemäße Vorrichtung sowohl in der Prävention für verschiedene Erkrankungen einzusetzen (Risiko-Bestimmung für Folgeerkrankungen), als auch in Real-Time-Szenarien, in denen unmittelbar auf aktuell detektierte Muster reagiert wird.

Die PC-Software dient der Visualisierung, Auswertung, Dokumentation und patientenbezogenen Archivierung von Langzeituntersuchungen zur Diagnostik von beispielsweise Schlafstörungen, Herz-Kreislauferkrenungen, Diabetes. Dafür wird das System konfiguriert und die übertragenen Daten automatisch offline analysiert. Die Software erlaubt die Eingabe von Bemerkungen durch den Anwender. Eine manuelle Reklassifikation der Analyseergebnisse durch den Auswerter ist möglich.

Der Patient ist nach Einweisung durch Fachpersonal und anhand der Gebrauchsanweisung für den Patienten in der Lage, die Sensoren und das Gerät selbst anzulegen.

Die erfindungsgemäße Vorrichtung verarbeitet und speichert alle gemessenen Signale auf der integrierten Speichereinheit, z.B. CompactFlash® Card. Ausgelesen werden die Daten entweder über ein USB-Kabel oder durch Auslesen der CompactFlash® Card mit einem Lesegerät. Die erfindungsgemäße Vorrichtung kann im stationären Betrieb die erfassten Daten online entweder drahtlos oder kabelgebunden an die Software übertragen, wo die Daten zusätzlich gespeichert werden.

Bei der Online-Überwachung mit der erfindungsgemäßen Vorrichtung sind in Kliniken vorhandene Netzwerke einsetzbar. Gehen Daten z.B. bei Verlassen des Untersuchungsraumes verloren, können diese Daten mit den auf der CompactFlash® Card gespeicherten Daten ergänzt werden. Die erfindungsgemäße Vorrichtung wird durch einen wechselbaren Akkupack versorgt, so dass diese unabhängig vom Netz ist. Durch einen Akkutausch gehen keine gespeicherten Messwerte verloren. Das Gerät kann auch dauerhaft mit dem Datenübertragungskabel versorgt und betrieben werden.

Die erfindungsgemäße Vorrichtung besitzt einen optionalen Lagesensor. Der Sensor registriert, ob und wann der Patient auf dem Bauch, dem Rücken oder auf der Seite liegt. Ebenso besitzt das Gerät optional einen im Gehäuse integrierten Effort-Sensor. Die Integration reduziert die Reinigung und erhöht die Lebensdauer des Sensors.

Mit Hilfe einer Drucktaste kann ein Sensortest/Impedanzcheck ausgelöst werden.

Mit Hilfe von Leuchtdioden oder eine Anzeige über ein integriertes Display kann bei einem Sensortest / Impedanzcheck festgestellt werden, ob bzw. welche Elektrode gut oder schlecht appliziert ist.

Zudem zeigt die erfindungsgemäße Vorrichtung durch eine gelbe Leuchtdiode im Akkupack neben dem Batteriesymbol oder durch ein Symbol auf dem integrierten Display an, ob der Akku momentan geladen wird. Der Ladezustand kann auch über die Software erfragt werden, da eine Kapazitätsüberwachung im Akku integriert ist.

Über das USB-Kabel oder Über die Konverterbox, in die eine galvanische Trennung integriert ist, können die gespeicherten Daten an den PC übertragen werden. Über die Konverterbox kann auch der Akku mit dem mitgelieferten Netzgerät geladen werden. Ein Akkumodul kann auch geladen werden, wenn es nicht im Gerät eingesetzt ist.

### Funktion der Software

Die während der Messung übertragenen Daten werden gespeichert und visualisiert. Die nach der Messung eingelesenen Daten werden nach Zeit- und Wertekriterien automatisch analysiert. Die Software kann beispielsweise folgende automatischen Analysen durchführen:
∘CWF-Analyse
∘Schnarch-Analyse
∘Kardiorespiratorische Analyse
∘Arousal Analyse
∘Schlafstadienanalyse

Auf der Grundlage der Analyseergebnisse und der dargestellten Signale können die vorliegenden Ergebnisse nach definierbaren Kriterien bewertet werden.

### Funktion der optionalen Sensoren

### Atemfluss-Schnarch-Nasenbrille

Die Atemfluss-Schnarch-Nasenbrille erfasst in Verbindung mit dem in die erfindungsgemäße Vorrichtung integrierten Drucksensor den Atemfluss sowie das Schnarchen. Die Inspiration wird über den erzeugten Unterdruck registriert, die Exspiration über den erzeugten Überdruck. Schnarchen erzeugt Druckschwankungen in den Nasenöffnungen, die ebenfalls registriert werden. Die Druckmessung reagiert bei geschlossenem Mund sensibler auf geringe Flusslimitierungen als die thermische Messung. Sie ist unabhängig von der Umgebungstemperatur und ermöglicht zusätzlich die visuelle Beurteilung der zeitlichen Flusskontur. Bei Mundatmung können die Signale abgeschwächt sein. Alternativ erfolgt deshalb der gleichzeitige Einsatz des Atemfluss-Mund-Sensors.

### Pulsoximetrie-Sensor

Über den Pulsoximetrie-Sensor werden die pulsoximetrischen Signale, die Sauerstoffsättigung des Blutes, die Pulsfrequenz und ein CWF-Signal gemessen.
∘Die Hauptbestandteile des Sensors sind zumindest zwei Leuchtdioden und eine Empfängerdiode.
∘Für jede Pulswelle werden mehrere SpO2-Werte bestimmt (Split-Pulswave-Algorithmus).

Die gemessenen Pulsfrequenzveränderungen entsprechen den Herzfrequenzveränderungen, die durch ein schlafbezogenes Apnoesyndrom ausgelöst wurden, hinreichend genau.

Mit Hilfe der Photoplethysmographie wird die Veränderung der Pulswelle, insbesondere die Amplitude der Pulswelle, ermittelt.

Die erfindungsgemäße Vorrichtung errechnet zu jedem erfassten Sauerstoffsättigungswert einen Qualitätsindex, welcher die Güte bzw. die Genauigkeit des gemessenen SpO2-Wertes kennzeichnet.

Wird das Signal durch Bewegungen gestört, ist die Anzahl der Werte gering. Bei störungsfreien Signalen liegt eine hohe Anzahl von Werten vor. Dementsprechend erzeugt ein gestörtes Messsignal einen niedrigen Qualitätswert, ein ungestörtes Messsignal hat einen hohen Qualitätswert zur Folge. Das Qualitätssignal nimmt Werte zwischen 0 und 100% an. Bei der Beurteilung von SpO2-Langzeitmessungen kann das Qualitätssignal hilfreich sein, denn es lässt auf Artefakte schließen, die während der Messung auftraten.

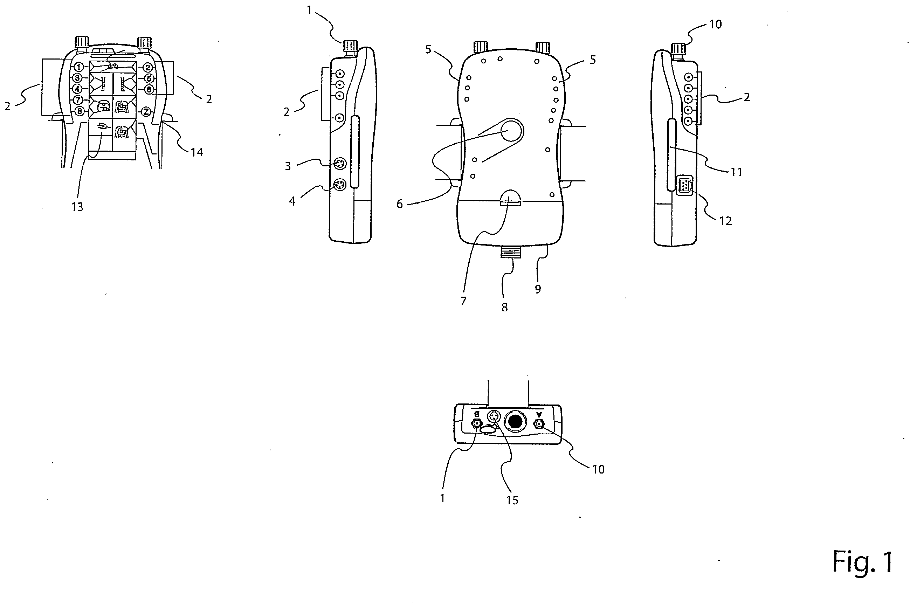

Fig. 1 zeigt ein Mobilteil mit einem Druckanschluß (1) zur Verbindung mit einem Druckmeßschlauch, Elektrodenanschlüssen (2), einem RIB (3) sowie einem Anschluß (4) für einen nicht dargestellten Abdomen-Sensor (35). Es sind ebenfalls nachfolgend noch näher erläuterte LEDs (5), eine Taste (6) sowie eine Akku-Verriegelung (7) zu erkennen. Des weiteren sind ein Anschluß (8) für ein Lade-/Datentransferkabel sowie ein Akku (9) zu erkennen. Vorzugsweise ist ein zweiter Druckanschluß (10) vorgesehen. Die Funktionalität wird durch einen Thoraxsensor (11) sowie einen Anschluß (12) für einen Pulsoximetrie-Sensor weiter gesteigert.

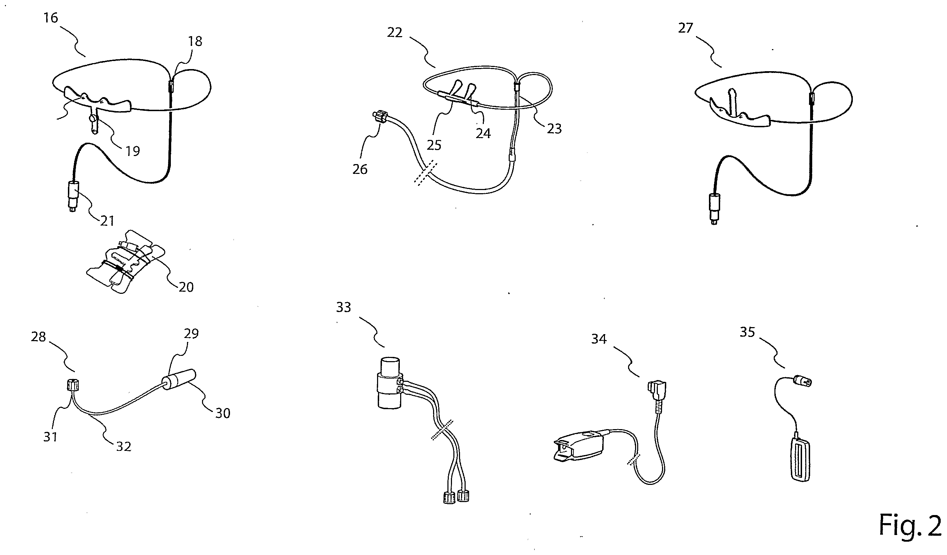

Im Bereich einer Rückseite des Mobilteiles ist eine Einlegekarte (13) mit Applikationsorten vorgesehen. Darüber hinaus sind eine Z-Elektrode (14) sowie ein Anschluß (15) zu erkennen. Der Anschluß (15) dient zur Verbindung mit einem in Fig. 2 dargestellten Atemfluß-Schnarch-Sensor (16) oder einem Atemfluß-Mund-Sensor (27). Der Anschluß (1) ist zur Verbindung mit einer Atemfluß-Schnarch-Nasenbrille (22) oder einem Druckmeß-Schlauch (28) vorgesehen. Die Anschlüsse (1, 10) dienen gemeinsam zur Verbindung mit einem Pneumo-T-Adapter (33) .

Fig. 2 zeigt zusätzlich zu den vorstehend bereits erwähnten Sensoren zum Anschluß an das Mobilteil Sensorperlen (17), eine Hülse (18), ein Mikrofon (19), eine Trägerplatte (20) sowie einen Sensorstecker (21). Ebenfalls sind eine Hülse (23), Kanülen (24), eine Lasche (25) sowie ein Anschluß (26) der Atemfluß-Schnarch-Nasenbrille (22) dargestellt.

Der Druckmeß-Schlauch (28) umfaßt ein Verbindungsstück (29), einen Verbindungsschlauch (30), einen Kunststoffschlauch (32) sowie ein Gewinde (31) für einen CPAP-Anschluß. Als weitere Sensoren sind ein Pulsoximetrie-Sensor (34) sowie ein Abdomensensor (35) abgebildet.

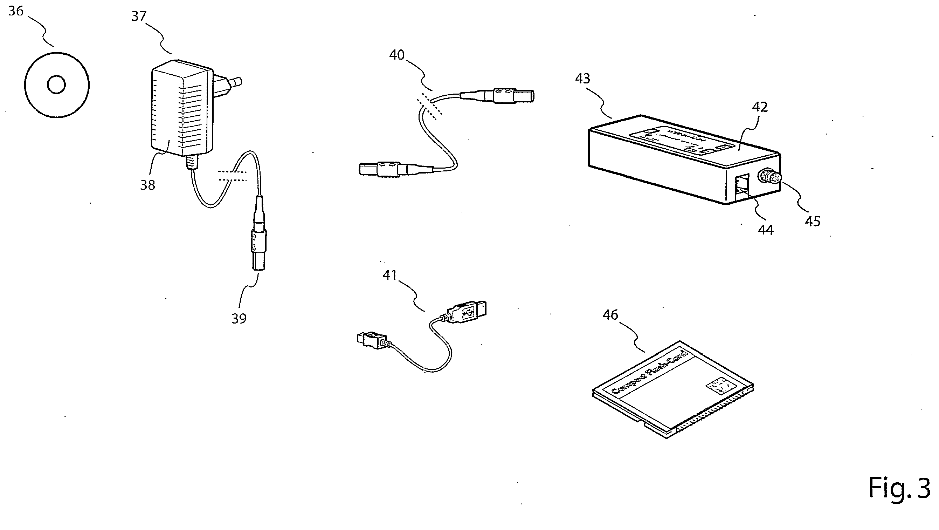

Fig. 3 zeigt Komponenten für den Datentransfer:
Das Mobilteil wird typischerweise an eine Auswertungseinrichtung angeschlossen, die als ein Personalcomputer ausgebildet sein kann. Die Auswertungseinrichtung umfaßt hierbei ein CD-ROM-Laufwerk mit CD (36), ein Ladegerät (37) mit Netzgerät (38) und Stecker (39), ein Lade-Daten-Transferkabel (40) und ein USB-Kabel (41). Eine Konverterbox (42) ist mit einer Buchse (43) für das Lade-Daten-Transferkabel (40), einer USB-Buchse (44) und einer Ladegerät-Buchse (45) ausgestattet. Eine Datenübertragung vom Mobilteil zur Auswertungseinrichtung kann auch direkt durch Umstecken einer Speicherkarte (46) erfolgen.

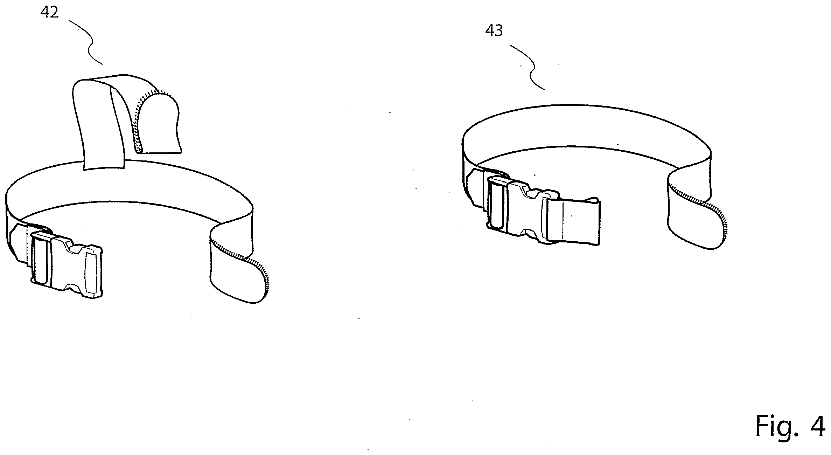

Fig. 4 zeigt zur Unterstützung einer mobilen Anwendung einen Gerätegurt (47) und einen Abdomengurt (48). Mit diesen wird die erfindungsgemäße Vorrichtung am Anwender befestigt. Der Gurt wird mit der Schnalle geschlossen. Durch Verstellen der Klettbänder kann der Gurt dem Körperumfang angepasst werden. Der Gurt besteht aus einem hautfreundlichen elastischen Flauschband.

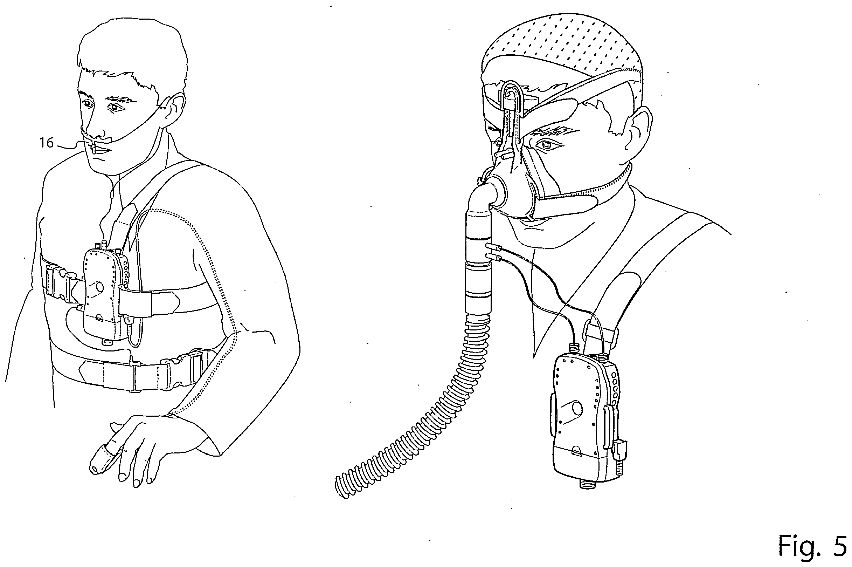

Fig. 5 zeigt im linken Zeichnungsteil eine Anwendung mit einem Atemfluß-Schnarch-Sensor (16) und im rechten Zeichnungsteil eine Anwendung zur Druckmessung im Bereich einer Beatmungsmaske. Die Vorrichtung ist am Anwender befestigt mit einem Pulsoximetriesensor und einem Atemfluss-Schnarch-Sensor beziehungsweise mit einem Pneumo-T-Adapter an einen Beatmungsschlauch angeschlossen.

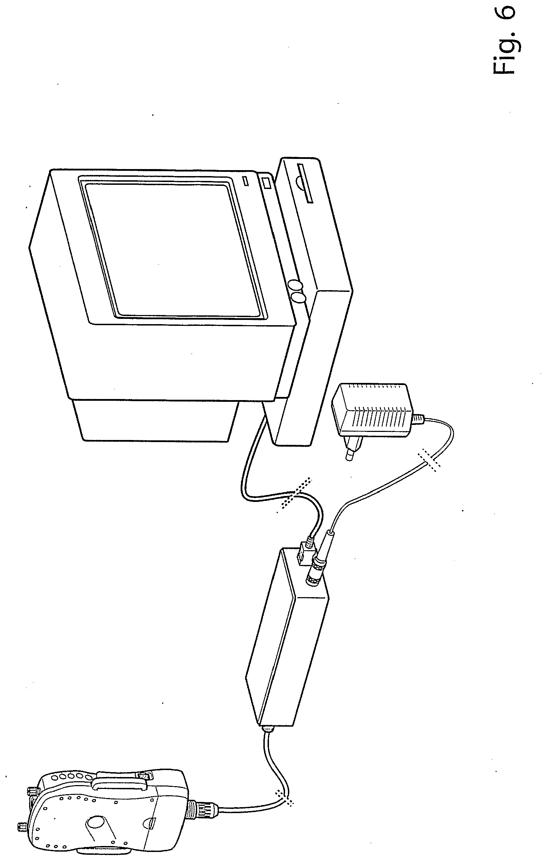

Fig. 6 zeigt eine angeschlossene Vorrichtung an eine Auswertungseinrichtung, die hier als ein Personalcomputer ausgebildet ist. Über ein medizinisch zugelassenes Netzgerät mit Kombikabel zur Ladung des Akkus und zur Datenübertragung der gespeicherten Daten über eine galvanisch getrennte USB-Schnittstelle der Konverterbox werden Daten an einen PC übertragen. Die Konverterbox dient zur kabelgebundenen Datenübertragung der im Gerät gespeicherten Daten. Die Datenübertragung erfolgt galvanisch getrennt über eine USB-Schnittstelle. Gleichzeitig wird die erfindungsgemäße Vorrichtung über das Netzgerät geladen, bzw. dauerhaft mit Strom versorgt. Auf dem PC läuft die PC-Software. Die PC-Software dient der Erfassung, Speicherung, Verarbeitung, Visualisierung, Auswertung, Dokumentation und der Archivierung von patientenbezogenen Biosignalen. Dies dient zur Unterstützung der Diagnosefindung, Therapieeinstellung und Therapiekontrolle von Schlafstörungen.

Über den Pulsoximetrie-Sensor (34) werden die pulsoximetrischen Signale, die Sauerstoffsättigung des Blutes und die Pulsfrequenz Ihres Patienten gemessen. Die Hauptbestandteile des Sensors sind zumindest zwei Leuchtdioden und eine Empfängerdiode. Für jede Pulswelle werden beispielsweise mehrere SpO2-Werte bestimmt (Split-Pulswave-Algorithmus).

Die gemessenen Pulsfrequenzveränderungen entsprechen den Herzfrequenzveränderungen, die durch ein schlafbezogenes Apnoesyndrom ausgelöst wurden, hinreichend genau.

Optional werden Sensoren verwendet, die alternativ und / oder ergänzend eine Ermittlung der Konzentration an Hämoglobin (cHb), Oxyhämoglobin (HbO2), desoxygeniertem Hämoglobin (HbDe), Carboxyhämoglobin (HbCO), Methämoglobin (MetHb), Sulfhämoglobin(HbSulf), Bilirubin, Glucose ermöglichen. Dazu weisen die Sensoren zumindest eine Lichtquelle auf die alternativ und/oder ergänzend folgende Wellenlängen emittiert, ausgewählt aus der Gruppe:
150 nm ± 15%, 400nm ± 15%, 460 nm ± 15%, 480 nm ± 15%, 520 nm ± 15%, 550 nm ± 15%, 560 nm ± 15%, 606 nm ± 15%, 617 nm ± 15%, 620 nm ± 15%, 630 nm ± 15%, 650 nm ± 15%, 660 nm ±, 705 nm ± 15%, 710 nm ± 15%, 720 nm ±1 0%, 805 nm ± 15%, 810 nm ± 15%, 880 nm ± 15%, 890 nm, 905 nm ± 15%, 910 nm ± 15%, 950 nm ± 15%, 980 nm ± 15%, 980 nm ± 15%, 1000 nm ± 15%, 1030 nm ± 15%, 1050 nm ± 15%, 1100 nm ± 15%, 1200 nm ± 15%, 1310 nm ± 15%, 1380 nm ± 15%, 1450 nm ± 15%, 1600 nm ± 15%, 1650 nm ± 15%, 1670 nm ± 15%, 1730 nm ± 15%, 1800 nm ± 15%, 2100 nm ± 15%, 2250 nm ± 15%, 2500 nm ± 15%, 2800 nm ± 15%.

Die Vorrichtung errechnet zu jedem erfassten Sauerstoffsättigungswert einen Qualitätsindex, welcher die Güte bzw. die Genauigkeit des gemessenen SpO2-Wertes kennzeichnet. Wird das Signal durch Bewegungen gestört, ist die Anzahl der Werte gering. Bei störungsfreien Signalen liegt eine hohe Anzahl von Werten vor. Dementsprechend erzeugt ein gestörtes Messsignal einen niedrigen Qualitätswert, ein ungestörtes Messsignal hat einen hohen Qualitätswert zur Folge. Das Qualitätssignal nimmt Werte zwischen 0 und 100% an. Bei der Beurteilung von SpO2-Langzeitmessungen kann das Qualitätssignal hilfreich sein, denn es lässt auf Artefakte schließen, die während der Messung auftraten.

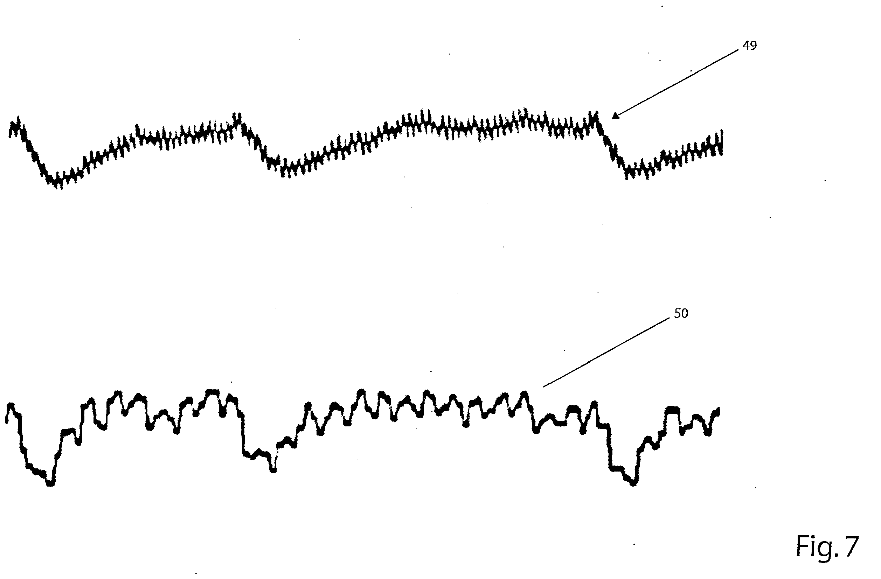

Fig. 7 zeigt oben das gemessenen Signal (49) als RohPlethysmogramm. Das Plethysmogramm (49) unterliegt Schwankungen ( = Fluktuationen). Aus dem Plethysmogramm (49) wird ein CWF-Signal (50) extrahiert. Das CWF-Signal (50) enthält Informationen über die Schwankungen (= Fluktuationen) des Plethysmogramms. Beispielsweise kann die Schwankung die Pulswellenamplitude darstellen. Es ist ebenfalls denkbar, das Integral des Plethysmogramms als CWF darzustellen. Erfindungsgemäß soll jegliche Schwankung des Plethysmogramms als CWF darstellbar sein.

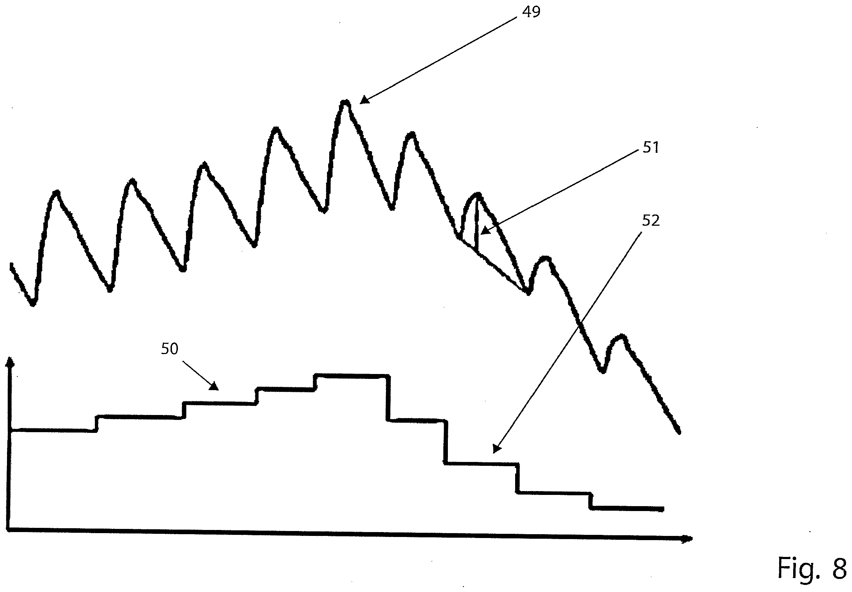

Fig. 8 zeigt eine mögliche Ausführungsform des CWF-Signals. Hier wurden die Amplituden (51) des Plethysmogramms (49) zur Ermittlung des CWF-Signals verwendet. Das CWF (50) stellt in diesem Fall die Amplitudenhöhe des Plethysmogramms (49) dar.

Es ist jedoch ebenfalls daran gedacht, die PTT oder andere Signale, die Schwankungen unterliegen, zur Ermittlung einer CWF zu nutzen.

Erfindungsgemäß können verschiedene Signale kombiniert werden, um relevante Schwankungen der Pulswelle zu detektieren und für die Auswertung heranzuziehen.

### Thoraxsensor und Abdomensensor

Thorax- und Abdomensensor dienen der Erfassung der thorakalen und abdominalen Atembewegungen.

Atembewegungen verursachen dabei wechselnde Zugspannungen auf die Messaufnehmer in den Befestigungsgurten. Die Messaufnehmer wandeln infolge des piezoelektrischen Effektes die Bewegungen in elektrische Signale um.

Der Abdomensensor erfasst, zusammen mit den Abdomengurten, die abdominalen Atembewegungen. Der Sensor besteht aus einem hautfreundlichen Kunststoff.

### Elektrophysiologische Signale

Die elektrophysiologischen Signale werden mittels Elektroden gemessen. Dazu können Goldnapf- oder Klebeelektroden verwendet werden.
∘Elektroenzephalogramm (EEG)
∘Elektrookulogramm (EOG)
∘Elektromyogramm (EMG)
∘Elektrokardiogramm (EKG)

### Atemfluss-Schnarch-Sensor (16)

Mit dem Atemfluss-Schnarch-Sensor werden nasaler und oraler Atemfluss und die Schnarchgeräusche erfasst. Die Sensorperlen bestehen aus Thermistoren. Sie erfassen den Atemfluss über die Temperatur der aus- und eingeatmeten Luft. Das Mikrofon registriert die Schnarchgeräusche des Patienten.

### Atemfluss-Mund-Sensor (27)

Mit dem Atemfluss-Mund-Sensor werden oraler Atemfluss bei der Diagnose mit der Atemfluss-Schnarch-Nasenbrille, Therapiekontrolle oder Therapieeinstellung erfasst. Die Sensorperlen bestehen aus Thermistoren. Sie erfassen den Atemfluss über die Temperatur der aus- und eingeatmeten Luft. Der Atemfluss-Mund-Sensor (27) wird bei der Diagnose zusammen mit der Atemfluss-Schnarch-Nasenbrille oder bei der Therapiekontrolle bzw. Therapieeinstellung zur Erfassung von Mundatmung zusammen mit dem Pneumo-T-Adapter (28) eingesetzt.

### Pneumo-T-Adapter (28)

Den Pneumo-T-Adapter wird zur Therapiekontrolle zusammen mit einer Nasalmaske eingesetzt. Durch ihn werden der Atemflow und das Schnarchen des Patienten während der Therapie registriert und der anliegende Therapiedruck in der Maske gemessen. Über die Druckmessschläuche werden in- und exspiratorische Druckschwankungen von der Maske zum Gerät geleitet. Das Ausatmen von Luft erzeugt einen geringen Überdruck, das Einatmen entsprechend einen Unterdruck. Aus den Druckunterschieden lassen sich die Atemzüge ableiten. Schnarchgeräusche werden über schnelle Druckveränderungen gemessen. Aus der statischen Komponente des Drucksignals wird der Therapiedruck abgeleitet. Der Pneumo-T-Adapter (28) wird bei der Therapieeinstellung und Therapiekontrolle zusammen mit xPAP-Geräten eingesetzt. Der Pneumo-T-Adapter kann zusammen mit dem Atemfluss-Mund-Sensor(27) eingesetzt werden, damit Mundatmung und Mundleckagen erkannt werden. Der Pneumo-T-Adapter hat einen Norm-Konus (ISO 22) zum Anschluss an Therapiemasken.

### EXG-Elektroden

Die mit den Elektroden erfasste Größe ist die Spannung. Gemessen wird dabei eine Spannungsdifferenz zwischen zwei Punkten des Körpers. Da die Messung an der Hautoberfläche nichtinvasiv erfolgt, sind die messbaren Spannungsdifferenzen sehr klein. Sie liegen beim EEG, EOG und EMG im Bereich *µ*V, beim EKG im Bereich mV.

### Bluetooth-USB-Adapter

Mit dem Bluetooth-USB-Adapter können von der erfindungsgemäßen Vorrichtung online Daten drahtlos empfangen oder übertragen werden, das Gerät konfiguriert, sowie eine Applikationskontrolle durchgeführt werden.

### Netzwerk USB-Server

Mit dem USB-Server kann die erfindungsgemäße Vorrichtung über ein Netzwerk betrieben werden. Über den USB-Server kann die erfindungsgemäße Vorrichtung in Verbindung mit dem Bluetooth-USB-Adapter Daten drahtlos empfangen, weiterhin kann das Gerät konfiguriert werden, sowie eine Applikationskontrolle durchgeführt werden. Ebenso kann die erfindungsgemäße Vorrichtung über die Konverterbox kabelgebunden angeschlossen werden.

### CompactFlash® Card-Lesegerät

Mit dem CompactFlash® Card-Lesegerät können die auf der CompactFlash® Card gespeicherten Daten von der erfindungsgemäßen Vorrichtung ausgelesen werden. Ebenfalls können über das CompactFlash® Card-Lesegerät die erfindungsgemäße Vorrichtung konfiguriert werden und/oder mehrere CompactFlash® Cards mit unterschiedlichen Konfigurationen angelegt werden.

### optionale Module

Eine ergänzende PC-Software ermöglicht das Auslesen und Darstellen der Therapiekontrolldaten und die Ferneinstellung aller genannten Therapiegeräte über die Software, sowie die PC-gestützte Auswertung von Titrationsdaten aus einem Beatmungs-Titrationsgerät.

### Kombination mit Therapiesystemen

Als Kontrollsystem kann die erfindungsgemäße Vorrichtung mit gängigen CPAP-, BiLevel- und APAP-Titrations-Heimbeatmungs -Therapiesystemen kombiniert werden. Die Koppelung der Systeme erfolgt schnell und einfach über den Pneumo-T-Adapter, der zwischen Schlauch und Maske gesteckt wird.

### Peripheriegeräte

∘USB-Port :von Windows unterstützt,
∘Anschlüsse: zumindest drei freie USB-Schnittstellen für den Anschluss von Kartenlesegerät, USB-Anschlusskabel zum Datalogger und Bluetooth®-USB-Adapter
∘Grafikkarte:von Microsoft Windows unterstützt, mind. Auflösung 1024x768, Farbtiefe 16 Bit
∘∘Monitor: 17" CRT-Monitor oder größer, oder 15" TFT-Monitor oder größer
∘Maus Windows-kompatible Maus
∘Drucker: von Microsoft Windows unterstützt
∘Netzwerk: Netzwerkkarte, 10/100 MBit (nur bei Verwendung des Netzwerk-USB-Servers)

### Software

Die Software dient zur Analyse und bietet alternative Auswertungsvorschläge an. Die Bewertung der automatisch erstellten Analyseergebnisse obliegt dem Arzt. Bei jedem neuen Programmieren der erfindungsgemäßen Vorrichtung wird die Uhrzeit im Grundgerät mit der Systemuhrzeit des PC's abgeglichen. Bei Unterbrechung der Datenübertragung zum PC werden die Messdaten im Gerät weiterhin gespeichert. In der Software werden die Signale als Nulllinie dargestellt. Alle Daten sind auslesbar. Wie die EMGs wird auch das EKG bipolar abgeleitet. Die polysomnographische Ableitung von der erfindungsgemäßen Vorrichtung lehnt sich an die Einthoven-Ableitung an. Die Bezugselektrode ist bei der erfindungsgemäßen Vorrichtung die Erdelektrode an einer beliebigen Körperstelle.

Mit Hilfe von Gurten, die in seitliche Ösen (a,b,c) des Gerätes geführt werden, kann die erfindungsgemäße Vorrichtung einem Patienten angelegt werden. Durch Klettbänder können die Gurte dem Körperumfang des Patienten angepasst werden. Der Gurt besteht aus einem hautfreundlichen elastischen Flauschband.

### Sensortest durchführen

Zur Überprüfung, dass alle Sensoren gut angeschlossen sind, kann nach dem Anlegen der Sensoren und der Geräte einen Test durchgeführt werden. Dazu wird die Taste (6) gedrückt.

| Vorgang | Gerät |
|---|---|
| Sensortest läuft | Während des Sensortests blinkt die LED des gerade getesteten Sensors schnell (4x pro Sekunde). |
| Sensortest ok | Die LED des entsprechenden Signals blinkt nach Beenden des Impedanztests nicht mehr: Impedanz der Elektrode < 5 kΩ in Ordnung, bzw. Sensorsignal vorhanden. |
| Sensortest mittel | Die LED des entsprechenden Signals blinkt nach Beenden des Impedanztests langsam: Impedanz der Elektrode < 10 kΩ nicht optimale, aber annehmbare Qualität. |

| | Grüne LEDs blinken langsam mit 0,5Hz. |
|---|---|
| Sensortest nicht ok | die LED des entsprechenden Signals blinkt nach Beenden des Impedanztests schnell: Impedanz der Elektrode > 10 kΩ bzw. kein Sensorsignal (Elektrode bzw. Sensor prüfen, inakzeptable Signalqualität). Grüne LEDs blinken schnell mit 1Hz. |

Nach erfolgreichem Anlegen aller konfigurierten Elektroden/Sensoren leuchtet an der erfindungsgemäßen Vorrichtung keine LED mehr. An der erfindungsgemäßen Vorrichtung gibt es dann keine Zustandsänderung der LED mehr, wenn im nächsten Schritt der Sensortest durch Schließen des Impedanzfensters in der Software beendet wird.

Beim Sensortest werden alle Kanäle, also auch Effort- und Pulsoximetriesensoren, sowie Thermistor und Nasenbrille auf Vorhandensein eines Signals geprüft. Wenn die LED aus ist, bedeutet dies: "Sensor ist angeschlossen und übermittelt ein (physiologisches) Signal".

Ein Impedanztest durchläuft immer einmal alle konfigurierten Kanäle und zeigt dann sein Ergebnis, bis das Fenster geschlossen oder ein neuer Test gestartet wird.

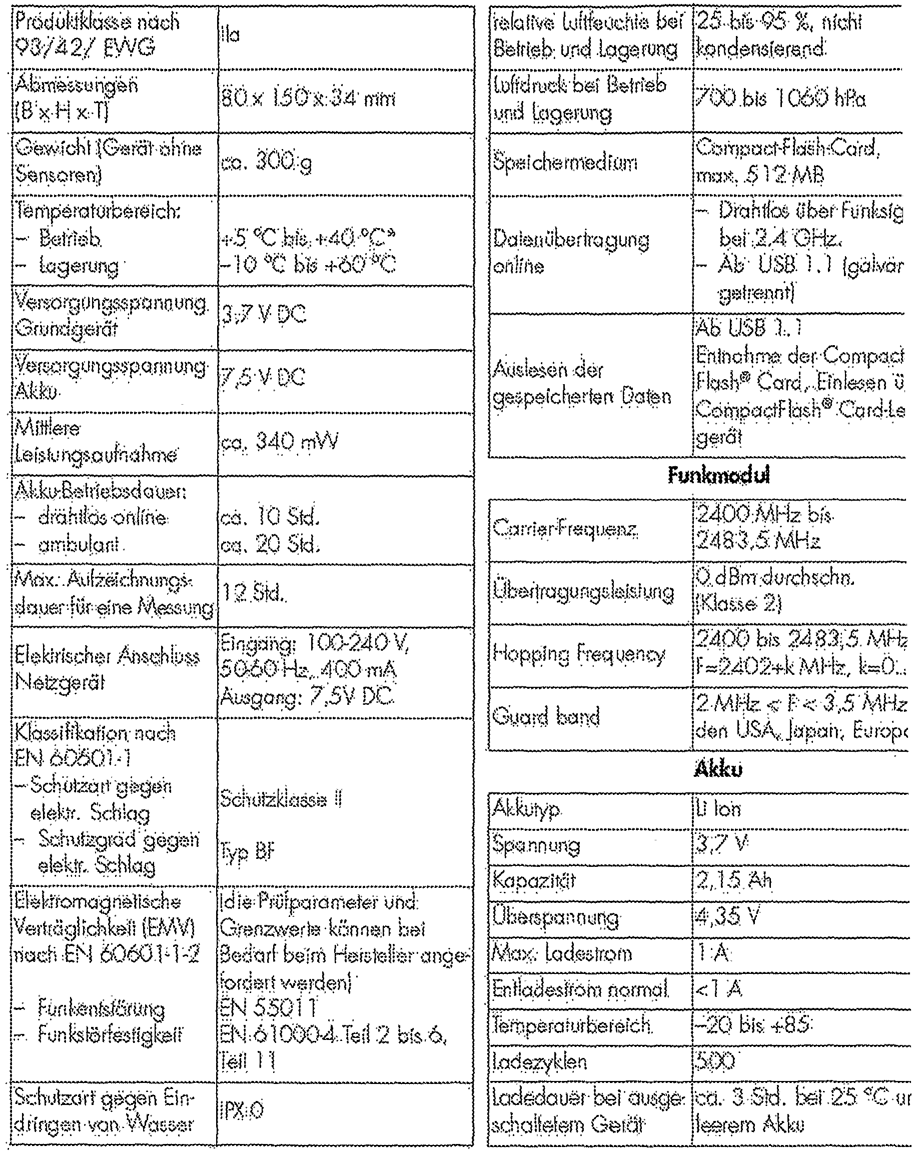

**Lage-Sensor**

| | |
|---|---|
| Lage-Sensor | im Gerät integrierter Sensor |
| Wertebereich | re. Seite, li. Seite, Bauch, Rücken, stehend |
| Genauigkeit | ca. 45° ± 15° |
| Lage | |
| CPAP- / BiPAP- / SmartPAP-Druck | |
| Messbereich | 0 bis 40 hPa |
| Genauigkeit | ±0,6 hPa |

**Pulsoximeter-Clipsensor**

| | |
|---|---|
| SpO2- Messbereich | 50 bis 100 % |
| SpO2- Genauigkeit | 70% < SpO2 < 100% besser als 2% Genauigkeit SpO2 |

**Pulsfrequenz**

| | |
|---|---|
| Messbereich | 30 bis 250 bpm |
| Puls Genauigkeit | ±1 bpm bis 2% vom Anzeigewert |
| Signalqualität | > 90 % |

**Atemfluss**

| | |
|---|---|
| Atemfluss-Schnarch-Sensor | 3 Thermistoren als Summensignal, keine Messfunktion bei Umgebungstemperaturen zwischen 33 - 38°C |
| Atemfluss-Schnarch-Nasenbrille | inspiratori- sche/exspiratorische Druckschwankungen |
| Atemfluss-Mund-Sensor | Ein Thermistor, keine Messfunktion bei Umgebungstemperaturen zwischen 33 - 38°C |

**Elektrohysiologische Signale**

| Kanal | EKG | EEG | EMG | EOG |
|---|---|---|---|---|
| Dynamikbereich (Physikalischer Wertebereich) | ± 5mV | ±500 *µ*V | ±250 *µ*V | ±500 *µ*V |
| Auflösung | 12 Bit | 12 Bit | 12 Bit | 12 Bit |
| Untere Grenzfrequenz | 0,16 Hz | 0,5 Hz | 2,7 Hz | 0,5 Hz |
| Obere Grenzfrequenz | 100 Hz | 100 Hz | 500 Hz | 100 Hz |
| Genauigkeit | ± 3% | ± 3% | ± 3% | ± 3% |
| Einstellbar sind | EMG, EOG, EEG, EKG | | | |
| Spezifikation | wie EMG, EOG, EEG, EKG | | | |
| Eingangsimpedanz | ca. 40 MΩ | | | |

### Technische Daten nichtmedizinischer Komponenten

**Flow Differenzdruck**

| | |
|---|---|
| Pneumo-T-Adapter | Konus nach Norm ISO 22 |
| Sensor | Differenzdruck: inspiratorische/exspiratorische Druckschwankungen |

**Effort Sensoren (Thorax, Abdomen)**

| | |
|---|---|
| Thorax Sensor | Im Gerät integrierter Sensor |
| Methode | Piezoelektrische Messung |

**Schnarchen**

| | |
|---|---|
| Atemfluss-Schnarch-Sensor | Integriertes Mikrofon |
| Atemfluss-Schnarch-Nasenbrille | Drucksensor |
| Pneumo-T-Adapter | Drucksensor |
| Methode | Log. Mittelwert des Schalldrucksignals (Mikrofon) bzw. der Druckschwankungen (Drucksensor) |

**Elektroden**

| | |
|---|---|
| Berührungssi-chere Steckverbinder, nach | 1,5 mm |

DIN 42802

Die Ausführungsbeispiele, die nicht unter den Schutzumfang der beigefügten Ansprüche fallen, dienen lediglich zu illustrativen Zwecken und sind nicht Gegenstand der gegenwärtigen Erfindung. Die Erfindung wird durch die folgenden Ansprüche definiert:

## Patentansprüche

1. Vorrichtung zur Bestimmung eines Vergleichswertes von Biodaten oder Körperparametern eines Lebewesens zur Ermittlung eines individuellen Risikos, bestehend aus einem tragbaren Grundmodel mit
mindestens einem Sensor (11, 14, 16, 17, 19, 22, 27, 28, 34, 35) zur nicht invasiven Messung von mindestens zwei Signalen ausgewählt aus der Gruppe: CWF (50), Sp02, Pulsfrequenz, Herzfrequenz, PTT und
mit einer an den Sensor (11, 14, 16, 17, 19, 22, 27, 28, 34, 35) angeschlossenen Auswertungseinrichtung, wobei die Auswertungseinrichtung wenigstens einen Analysator aufweist, der durch Signalanalyse definierbare Signalbereiche ermittelt und
mit einer Schnittstelle für die Steuerung eines Therapiegerätes und
mit einer USB Schnittstelle und
mit einer integrierten Speichereinheit und
mit einer Vergleichseinrichtung die die Signalbereiche unter Berücksichtigung weiterer Parameter wie bestehende Biodaten oder Körperparamter des Lebewesens und/oder statische Datensätze abstimmt und das Ergebnis als Index-Wert ausgibt wobei der Sensor das Rohplethysmogramm als ein Signal aufzeichnet, aus welchem die Auswertungseinrichtung einen CWP extrahiert, welcher über die Zeit betrachtet als CWF-Signal berechnet wird, sowie dass das CWF-Signal für die Gerätesteuerung ausgewertet wird und wobei alle gemessenen Signale verarbeitet und auf der integrierten Speichereinheit gespeichert werden, die Daten über ein USB-Kabel ausgelesen werden und dass die Übertragung der Daten über die USB-Schnittstelle erfolgt.

2. Vorrichtung nach Anspruch 1, **dadurch gekennzeichnet, dass** die Parameter der Biodaten des Lebewesens wie Medikation, Krankheitshistorie, Alter, Geschlecht, Diagnosedaten, beinhalten.

3. Vorrichtung nach Anspruch 1 oder 2, **dadurch gekennzeichnet, dass** die statischen Datensätze aus Gruppen von Lebewesen, wie Medikation, Alter, Geschlecht, Diagnosedaten und dergleichen, zusammenstellbar sind.

4. Vorrichtung nach einem der Ansprüche 1 bis 3, **dadurch gekennzeichnet, dass** die Vergleichseinrichtung mit Peripheriegeräte, wie Rechner, Drucker bzw. Sichtgeräte koppelbar ist.

5. Vorrichtung nach einem der Ansprüche 1 bis 4, **dadurch gekennzeichnet, dass** die Auswertungseinrichtung zur Ermittlung eines CWP aus mindestens einem erfassten Messsignal ausgebildet ist.

6. Vorrichtung nach Anspruch 5, **dadurch gekennzeichnet, dass** die Auswertungseinrichtung zur Ermittlung einer CWF (50) aus mindestens einem der erfassten Messsignale ausgebildet ist.

7. Vorrichtung nach einem der Ansprüche 1 bis 6, **dadurch gekennzeichnet, dass** die Auswertungseinrichtung zur Ermittlung einer CWF (50) aus dem CWP ausgebildet ist.

8. Vorrichtung nach einem der Ansprüche 1 bis 7, **dadurch gekennzeichnet, dass** die Auswertungseinrichtung zur Ermittlung der CWF (50) sowohl aus mindestens einem erfassten Messsignal und dem CWP ausgebildet ist.

## Claims

1. Apparatus for determining a comparison value of biodata or body parameters of a living being for establishing an individual risk, consisting of a portable basic model having at least one sensor (11, 14, 16, 17, 19, 22, 27, 28, 34, 35) for a non-invasive measurement of at least two signals selected from the group: CWF (50), SpO₂, pulse rate, heart rate, PTT, and having an evaluation device connected to the sensor (11, 14, 16, 17, 19, 22, 27, 28, 34, 35), wherein the evaluation device has at least one analyser, which establishes definable signal regions by signal analysis, and having an interface for controlling a therapy appliance and having a USB interface and having an integrated storage unit and having a comparison device which adjusts the signal ranges taking account of further parameters such as existing biodata or body parameters of the living being and/or static data records and outputs the result as an index value, wherein the sensor records the raw plethysmogram as a signal from which the evaluation device extracts a CWP, which, considered over time, is calculated as the CWF signal, and in that the CWF signal is evaluated for the appliance controller and wherein all measured signals are processed and stored on the integrated storage unit, the data are read out via a USB cable and in that the transmission of the data is implemented via the USB interface.

2. Apparatus according to Claim 1, **characterized in that** the parameters of the biodata of the living being contain such as medication, history of illnesses, age, sex, diagnostic data.

3. Apparatus according to Claim 1 or 2, **characterized in that** the static data records are assemblable from groups of living beings, such as medication, age, sex, diagnostic data and the like.

4. Apparatus according to any one of Claims 1 to 3, **characterized in that** the comparison device is coupleable to peripheral appliances such as computers, printers or monitors.

5. Apparatus according to any one of Claims 1 to 4, **characterized in that** the evaluation device is embodied to establish a CWP from at least one captured measurement signal.

6. Apparatus according to Claim 5, **characterized in that** the evaluation device is embodied to establish a CWF (50) from at least one of the captured measurement signals.

7. Apparatus according to any one of Claims 1 to 6, **characterized in that** the evaluation device is embodied to establish a CWF (50) from the CWP.

8. Apparatus according to any one of Claims 1 to 7, **characterized in that** the evaluation device is embodied to establish the CWF (50) from both at least one captured measurement signal and the CWP.

## Revendications

1. Dispositif destiné à déterminer une valeur comparative de données biologiques ou de paramètres corporels d'un organisme vivant pour déterminer un risque individuel, constitué d'un modèle de base portable muni d'au moins un capteur (11, 14, 16, 17, 19, 22, 27, 28, 34, 35) pour la mesure non invasive d'au moins deux signaux choisis dans le groupe : CWF (50), Sp02, fréquence du pouls, fréquence cardiaque, PTT et
muni d'un dispositif d'analyse connecté au capteur (11, 14, 16, 17, 19, 22, 27, 28, 34, 35), dans lequel le dispositif d'analyse comporte au moins un analyseur qui détermine des plages de signaux définissables par analyse de signaux, et
muni d'une interface pour la commande d'un appareil de thérapie et
d'une interface USB et
d'une unité de stockage intégrée et
d'un dispositif de comparaison qui harmonise les plages de signaux en tenant compte d'autres paramètres tels que des données biologiques existantes ou des paramètres corporels de l'organisme vivant et/ou des ensembles de données statiques et qui délivre le résultat sous la forme d'une valeur d'indice,
dans lequel le capteur enregistre le pléthysmogramme brut sous la forme d'un signal à partir duquel le dispositif d'analyse extrait un CWP, qui est calculé au cours du temps sous la forme d'un signal CWF, et en ce que le signal CWF est analysé pour la commande de l'appareil, et
dans lequel tous les signaux mesurés sont traités et stockés sur l'unité de mémoire intégrée, les données sont lues par l'intermédiaire d'un câble USB et en ce que les données sont transmises par l'intermédiaire de l'interface USB.

2. Dispositif selon la revendication 1, **caractérisé en ce que** les paramètres des données biologiques de l'organisme vivant comprennent tels que les traitements médicaux, les antécédents pathologiques, l'âge, le sexe, des données diagnostiques.

3. Dispositif selon la revendication 1 ou 2, **caractérisé en ce que** les ensembles de données statistiques peuvent être établis à partir de groupes d'êtres vivants, et comprennent des paramètres tels que les traitements médicaux, l'âge, le sexe, des données diagnostiques et autres.

4. Dispositif selon l'une des revendications 1 à 3, **caractérisé en ce que** le dispositif de comparaison peut être couplé à des dispositifs périphériques tels que des ordinateurs, des imprimantes ou des appareils d'affichage.

5. Dispositif selon l'une des revendications 1 à 4, **caractérisé en ce que** le dispositif d'analyse est conçu pour déterminer un CWP à partir d'au moins un signal de mesure acquis.

6. Dispositif selon la revendication 5, **caractérisé en ce que** le dispositif d'analyse est conçu pour déterminer une CWF (50) à partir d'au moins l'un des signaux de mesure acquis.

7. Dispositif selon l'une des revendications 1 à 6, **caractérisé en ce que** le dispositif d'analyse est conçu pour déterminer une CWF (50) à partir du CWP.

8. Dispositif selon l'une des revendications 1 à 7, **caractérisé en ce que** le dispositif d'analyse destiné à déterminer la CWF (50) est réalisé à la fois à partir d'au moins un signal de mesure acquis et du CWP.
